# EUROPEAN PATENT SPECIFICATION

(11) **EP 4 574 117 B1**
(45) Date of publication and mention of the grant of the patent: **12.08.2026**
(21) Application number: 23219550.3
(22) Date of filing: 21.12.2023
(51) Int. Cl.: A61F 13/42

(54) **ABSORBENT ARTICLE WITH LEAKAGE PROBABILITY ALGORITHM**
SAUGFÄHIGER ARTIKEL MIT LECKAGEWAHRSCHEINLICHKEITSALGORITHMUS
ARTICLE ABSORBANT AVEC ALGORITHME DE PROBABILITÉ DE FUITE

(43) Date of publication of application: 25.06.2025
(73) Proprietor: Ontex BV, 9255 Buggenhout (BE); Ontex Group NV, 9320 Erembodegem (BE)
(72) Inventor: Hellmold, Jens, 59269 Beckum (DE); PEPERMANS, Manu, 2018 Antwerpen (BE)
(74) Representative: Macchetta, Andrea

(56) References cited:
- WO-A1-2023/232263
- US-A1- 2012 256 750
- US-B2- 9 931 251

## Description

### TECHNICAL FIELD

The disclosure relates to absorbent articles such as disposable absorbent articles, preferably selected from the group consisting of diapers (whether for baby, youth, or adults), pants (whether for baby, youth or adults), briefs, pantyliners, slips, sanitary napkins or towels, sanitary napkins for light incontinence, and the like; preferably selected from diapers and pants.

More particularly the invention pertains to diapers or pants, components thereof, and process of making, comprising an wetness monitoring system capable of automatically monitoring the distribution of exudates within the diaper or pant warn by a subject, and automatically providing a warning to a care giver when the risk of leakage is elevated and said diaper or pant should be replaced.

### BACKGROUND

Disposable diapers conventionally include a chassis having a liquid permeable topsheet, a liquid impermeable backsheet and an absorbent structure sandwiched between the topsheet and backsheet. The chassis has a front body panel which, in use, extends over the stomach and front hip area of the user, and a rear body panel which, in use, extends over the back and the rear hip area of the user. Each of the body panels has a waist portion such that, when the diaper is fastened around the waist of the user, the waist portions provide a continuous encirclement of the user. In order to fasten the diaper around the waist of a user, a fastening system comprising fastening tabs is commonly employed. Fastening tabs may be provided on side panels which extend from lateral side edges of the diaper chassis.

Disposable pants have a similar construction but typically comprise front and back elasticized belts at either end of the absorbent structure and are sealed together at lateral side seams to form an underwear-resembling product that can be worn by a subject by pulling it up over the legs and may be removed either by pulling it down in the opposite direction of by tearing the side seams.

Inherent with the use of such absorbent articles, eventually one or more wetness and/or exudate events will occur and thus need for change of the absorbent article arises. Particularly in home care and/or institutions, handling elderly with incontinence problems, knowing when to change the diaper/pant of a patient is of the utmost importance. Indeed, changing the diapers too early results in unnecessary cost and waste and changing too late results in further cleaning costs and time, and uncomfort or discomfort to the patient. This problem is exacerbated by motion, indeed taking into account that a subject wearing the article may move into different positions the saturation thereof and/or risk of leakage may vary considerably based on the position and time the subject remains in that position during and/or after one or more wetness/exudate events.

Absorbent articles possessing different types of detecting means are known, and help to alert a user or caregiver to a change within the article (e.g. a soiling event). Such detecting means allow the user or caregiver to readily determine whether or not an absorbent article needs to be changed, without the need for close inspection or removal of the article.

Commonly known detecting means which can be incorporated into absorbent articles are chemical substances which alter their form or nature upon contact with liquid. For example, an indication that an absorbent article is soiled can arise from colour changes or the appearance or disappearance of an element on the absorbent article. Such detecting means are useful in certain situations, but less in institutions such as child-care centres, care centers for the elderly or hospitals where the status of a large number of wearers must be monitored; often by a limited staff.

Determining whether the absorbent article is soiled or not still requires the wearer to be disturbed, as the coloured element must still be visible to the caregiver. This often requires that the wearer be moved, and their clothes removed or adjusted.

EP3415130A1 and WO2018229017 refers to an absorbent article, components and process therein using detecting means that provides the advantages of electrical detection, yet which is cost-effective, simple to manufacture and readily disposable. Nevertheless, the solution implemented in EP3415130A1 does not allow to identify the exact zones or locations at which the absorbent article is wet.

EP2019659B1 provides for a moisture monitoring system for monitoring wetness in one or more absorbent articles, includes an input for receiving one or more sensor signals indicative of a presence of wetness in an absorbent article, a processor for processing the one or more sensor signals and for performing an analysis of the signals to characterise wetness events occurring in an absorbent article and user interface for communicating with a user of the system. A mathematical model may be used to characterise wetness events, receiving as inputs variables derived from sensor signals and optionally, patient and demographic data. The mathematical model can be configured and/or re-configured by an algorithm utilising observation data obtained while monitoring a patient for wetness.

EP3427709A1 relates to a Wetness monitoring apparatus for incontinence surveillance, comprising - One or more sensing elements, said sensing elements comprising one or more conductive paths, said conductive paths being printed of one or more conductive copolymers, - an absorbent article having an absorbent component, wherein said sensing elements being printed onto one or more components of said absorbent article, - a readout device being adapted to detect changes in the electrical properties of said sensing elements due to moisture, fluids or the like at least in the vicinity of said sensing elements, and - a docking component for removably coupling said readout device to said absorbent article, said readout device being electrically connected to said sensing elements when coupled via docking component. Wetness detection devices and methods with multiple electrodes or conductors are shown also in WO 2023232263 and US 2012256750 A1.

There is however still a need for absorbent articles comprising detecting means providing on-time alerts based on the risk of exudate leakage from the absorbent article, without the need for volume determination and observation data. There is the desire for a simple system that allows for accurate alarms for assisting changeovers of patients without monitoring amount of liquid unlike the prior art.

### SUMMARY

In a first aspect the disclosure relates to a method for monitoring wetness events in an absorbent article comprising the steps of: providing an absorbent article comprising a liquid permeable topsheet, a substantially liquid impermeable backsheet, an absorbent core positioned between said topsheet and backsheet. The absorbent article further comprises a substrate adapted for incorporation into the absorbent article comprising a plurality of conductors and a plurality of electrodes arranged to undergo a change in electrical properties upon one or more wetness events. The method, upon a wetness event, comprises the step of determining the number of conductors triggered by said wetness event to cumulatively determine the number of activated electrodes. The cumulative number of activated electrodes is compared to a threshold value and an alarm is triggered when the number of activated electrodes is equal to or greater than the threshold value.

### BRIEF DESCRIPTION OF THE FIGURES

**FIG. 1a** is a plan view representation of an absorbent article according to the present invention.
**FIG. 1b** is a cross-section representation of an absorbent article according to the present invention.
**FIG. 2** is a schematic top view of an absorbent core comprising one or more channels.
**FIG. 3** is a schematic top view of a substrate according to an embodiment of the present invention.
**FIG. 4** depicts schematically a portion of an absorbent article and pocket according to an embodiment herein.
**FIG. 5** depicts a plan view representation of an absorbent article comprising a data processing module.
**FIG. 6** depicts the structure of a clip-on data processing module.
**FIG. 7** is a schematic top view of an absorbent article comprising a front waist barrier and/or back waist barrier.
**FIG. 8** is a schematic top view of an absorbent core comprising a first and a second eccentric detection zone.
**FIG. 9** is a sectional view along the longitudinal axis of an exemplary absorbent article comprising a FWB.
**FIG. 10** is a schematic top view representation of an absorbent article comprising 4 eccentric detection zones.
**FIG. 11** is a schematic top view of an absorbent core comprising a first and a second concentric detection zone.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-40% or less, or +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" or "substantially" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's garments or undergarments when the absorbent article is worn.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

The term "disposable" is used herein to describe absorbent articles that generally are not intended to be laundered or otherwise restored or reused as an absorbent article (i.e., they are intended to be discarded after a single use and, preferably, to be recycled, composted, or otherwise disposed of in an environmentally compatible manner.

A "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0,001 mm to more than about 0,2 mm and the come in several different forms such as short fibers (known as staple, or chopped), continuous single fibers (filaments or monofilaments), untwisted bundles of continuous filaments (tow), and twisted bundles of continuous filaments (yarn). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The term "joined" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element. The terms further included embodiments in which a pocket or other connector is formed in or attached to an area of the absorbent article. Further, these terms include configurations in which the elements are removably, or non-removably, joined, bonded, or attached. For example, wherein an element is described as "joined" within the configuration, it may be either removably joined or non-removably joined unless otherwise specified or evident from the context.

The terms "comprise", "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined below and glue enclosed within the core wrap.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

Use of the term "substrate" includes, but is not limited to, woven or nonwoven webs, porous films, ink permeable films, paper, composite structures, or the like.

"Wetness sensing tracks" as used herein refer to tracks that are suitable for wetness sensing but are not limited thereto, for example said tracks may further or alternatively sense other form of exudates that not necessarily result in wetness, such as stool detection. These can be configured to undergo changes in presence of certain substances, such as stool and /or cellulose fibers combined with superabsorbent polymers saturated with urine.

"Terminal" refers to the point at which a conductor from an electrical component, device or network comes to an end and provides a point of connection to external circuits. With this, terminals integrated into the clip-on data processing module as described herein are meant. These consist by preference of metal or a metal alloy, more preferably of a metal or metal alloy plated by a precious metal, even more preferably gold plated.

"Trigger" refers to a stimulus that elicits a reaction, a stimulus that causes a reaction. Within the context of the present invention, a wetness event can "trigger" an alarm.

"Threshold value" refers to a value that sets a limit or boundary, above or below which a different state or condition is observed. It is thus an amount, level or limit on a scale. When the threshold is reached, something else happens or changes.

"Theoretical miction point" refers to a theoretical point of miction located at a distance from the front edge of the absorbent core wherein this distance is 2/5 of the total length of the absorbent core.

The term "top sheet" or "topsheet" refers to a liquid permeable material sheet forming the inner cover of the absorbent article and which in use is placed in direct contact with the skin of the wearer. The topsheet is typically employed to help isolate the wearer's skin from liquids held in the absorbent structure. The topsheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The topsheet material may further be composed of two fibres, which may be bonded to each other in a bonding pattern. Further examples of top sheet materials are porous foams, apertured plastic films, laminates of nonwoven materials and apertured plastic films etc. The materials suited as topsheet materials should be soft and non-irritating to the skin and be readily penetrated by body fluid, e.g. urine or menstrual fluid. The inner coversheet may further be different in different parts of the absorbent article. The topsheet fabrics may be composed of a substantially hydrophobic material, and the hydrophobic material may optionally be treated with a surfactant or otherwise processed to impart a desired level of wettability and hydrophilicity.

The term "back sheet" or "backsheet" refers to a material forming the outer cover of the absorbent article. The backsheet prevents the exudates contained in the absorbent structure from wetting articles such as bedsheets and overgarments which contact the disposable absorbent article. The backsheet may be a unitary layer of material or may be a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapour to escape from the absorbent material, while still preventing liquids from passing there through. Examples of breathable backsheet materials are porous polymeric films, nonwoven laminates of spunbond and meltblown layers and laminates of porous polymeric films and nonwoven materials.

The term "electrode" refers to an electrical conductor used to make contact with a non-metallic part of a circuit (e.g. a semiconductor, an electrolyte, a vacuum or air). In the present invention the non-metallic part would be the absorbent material comprises exudates.

The term "activate" or "activated" refers to making something active or something that is made active. In the presented invention, an activated electrode refers to an electrode that has made contact with absorbent material comprising exudates and therefor measures a change in electrical properties.

The term "registered" (e.g. registered design) refers to, for example, a print design that varies along the length of a continuous web/film (and is repeated at intervals throughout) and that hence requires some form of recognition on where a processing action must happen, e.g. a cut, to ensure that consecutive discrete products formed from the continuous web/film each have the same print design.

The terms "wetness" and "wetness event" are to be understood as including human urination, defecation, and other bodily discharge events.

The term "concentric" refers to circles, arcs, or other shapes which share the same center, the larger often completely surrounding the smaller.

The term "eccentric" refers to circles, arcs or other shapes which do not share the same center, the larger often completely surrounding the smaller.

The values indicated herein are measured according to the methods indicated herein below, unless specified otherwise. All measurements are performed at 21±2°C. and 50±20% RH, unless specified otherwise. All measurements should be reproduced on at least 4 samples and the average value obtained indicated, unless otherwise indicated.

Embodiments of the articles according to the disclosure will now be described. It is understood that technical features described in one or more embodiments maybe combined with one or more other embodiments without departing from the intention of the disclosure and without generalization therefrom, especially when such combinations are explicitly or implicitly inferred.

### THE ABSORBENT ARTICLE

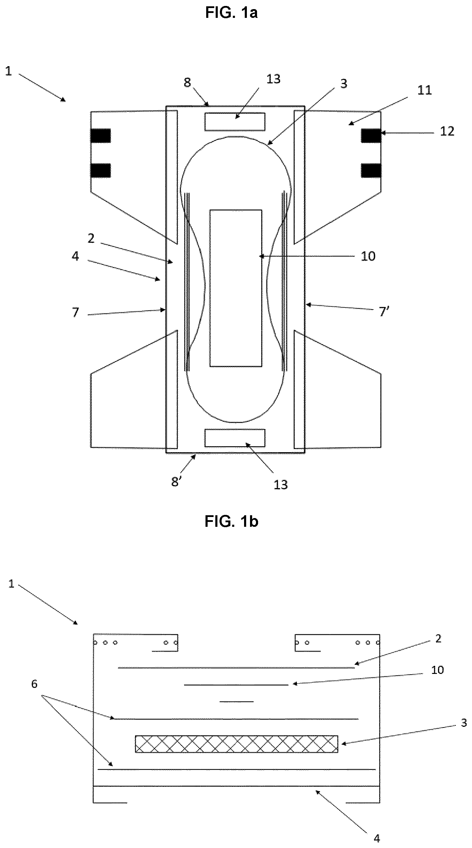

The absorbent article (1) (as exemplified in Figs. 1a-1b) is typically a disposable diaper, pad or pant, and suitable for detecting an exudate and/or wetness (i.e. urine and/or feces) event therein and/or risk of exudate leakage therefrom, said absorbent article (1) comprising: a liquid impermeable backsheet (4), a liquid permeable topsheet (2), and an absorbent core (3) interposed between said backsheet (4) and topsheet (2). Typically the backsheet (4), absorbent core (3) and topsheet (2) form a chassis of the absorbent article (1).

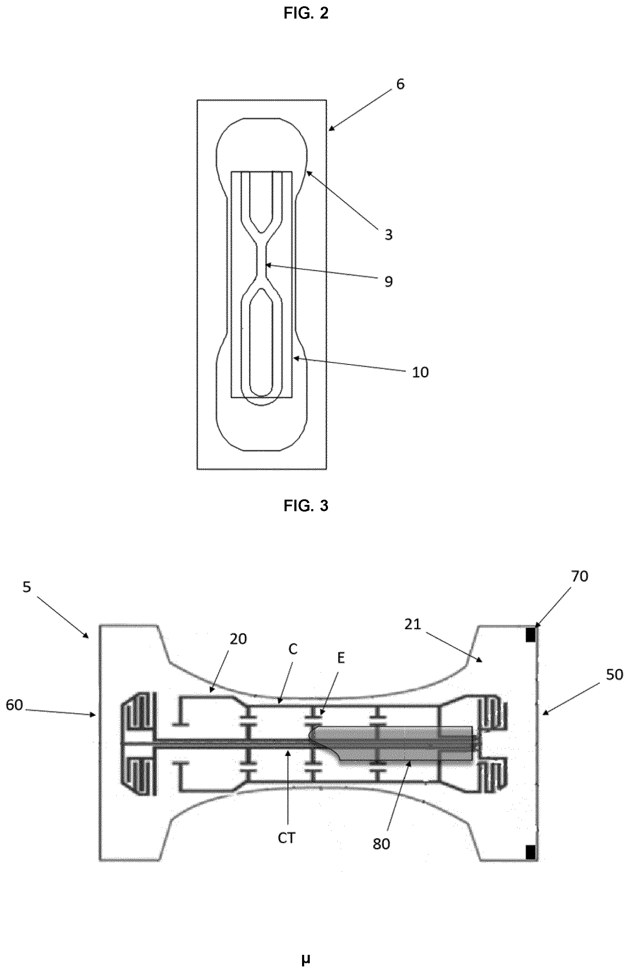

The absorbent core (3), as for example illustrated in Fig.1a, has a first and second longitudinal edge (7, 7') and a front and back transverse edge (8, 8'). The absorbent core (3) comprises absorbent material selected from the group consisting of cellulose fibers, superabsorbent polymers and combinations thereof, and the absorbent material is contained within at least one core wrap substrate (6) enclosing said absorbent material. The top layer of said core wrap (6) may be adhered to a bottom layer of said core wrap (6) to form one or more channels (9), as exemplified in Fig. 2, substantially free of said absorbent material. The core wrap substrate typically has hydrophilic properties.

The absorbent article (1) may further comprise additional components, as exemplied in Figs.1a-1b, that are common in the art and selected from the group consisting of: a liquid distribution layer (ADL) (10) positioned between the topsheet (2) and the absorbent core (3); elastic or non-elastic back ears (11) joined to the chassis at a position proximal to the second end of the substrate forming the backsheet (4); super absorbent polymer particles and/or fibers typically comprised within the absorbent core (3); cellulose fibers typically comprised within the absorbent core (3); fastening tapes (12) joined to the back ears (11) for adhesive and/or mechanical coupling with a garment facing surface of the backsheet (4) at a position proximal to the first end; elastic waistband (13) positioned proximal to the first and/or second ends; and combinations thereof.

When the absorbent article (1) is a pant, the absorbent article (1) may comprise a front belt positioned proximal to the first end and a rear belt positioned proximal to the second end, said belts being separated from each other by the chassis of the absorbent article and being directly joined to each other via two opposite side seams; said belts typically being elastic. When the absorbent article is a pad it may be designed as a two-piece system, where the second piece is an elastic pant (reusable or disposable) to hold the first piece - said absorbent article - securely in place.

The absorbent article (1) further comprises a substrate (5), as exemplified in Fig. 3, for monitoring and detecting the presence of wetness events therein and/or risk of exudate leakage therefrom. In a preferred embodiment, the backsheet (4) comprises said substrate (5). The substrate (5) comprises a conductive pattern (20) disposed on a first surface (21) capable of being arranged proximal to a body-facing side of the absorbent article (1), wherein said conductive pattern (20) can be brought in electrical communication with a data processing module (30), preferably a clip-on data processing module. Said conductive pattern (20) comprises a plurality of connecting tracks (CT), a plurality of conductors (C) and a plurality of electrodes (E) connected to said conductors (C) and wherein the conductive pattern is configured in a manner that addressing multiple combinations of said plurality of connecting tracks (CT) with corresponding terminals (T) of the data processing module (30), preferably a clip-on data processing module, result in multiple electrical circuit configurations for measuring resistance, impedance and/or capacitance therethrough.

In a preferred embodiment, the substrate (5) comprises a first end (50) corresponding to the front (or belly portion) of the absorbent article (1) and a second end (60) corresponding to the back of the absorbent article (1).

In a preferred embodiment, the connecting tracks (CT) are configured to allow an electrical current to flow from corresponding terminals (T) of the data processing module (30), preferably a clip-on data processing module, when connected thereto. Additionally or alternatively, said connecting tracks (CT) may also be configured to allow an electrical current to flow to, or from, corresponding terminals (T) of the data processing module (30), preferably a clip-on data processing module.

It may be beneficial that said connecting tracks (CT), extend substantially parallel to a length L of the substrate (5) and substantially parallel to a longitudinal axis (y-y) crossing a first end (50) and a second end (60) of the substrate (5). Moreover, the electrodes (E) are configured to undergo changes in presence of certain substances, such as stool and/or cellulose fibers combined with superabsorbent polymers saturated with urine.

Advantageously, it has been found that a substrate (5) comprising the above described conductive pattern (20) provides for accurate detection of exudates as well as being particularly suitable for fully scalable for in-line mass production at high speeds, without the need to change arrangement depending on product sizes. Further advantageously, the conductive pattern (20) allows for multiple usage of terminals (T) of the data processing module (30), preferably a clip-on data processing module, which in turn enables to detect wetness along different, specific zones of the absorbent article (1).

Preferably, the conductive pattern (20) comprises an electrically conductive material, and is preferably a printed conductive pattern, so that the distribution of said conductive pattern (20) (also referred to herein as sensor design) is registered and, hence, the sensor design is directly comprised on the substrate (5). This is advantageous, since further modifications as e.g. in order to characterize the absorbent product (1) (including for example information regarding the product size, absorbency of the core, colour of the product or the like), can be directly comprised within the printed conductive pattern (20). In addition to this, said sensor design may also comprise registration marks (70), as depicted in Fig. 3, arranged to provide a trigger to a registering device to carry out process steps.

Preferably, the printed conductive pattern (20), comprising the plurality of conductors (C) and the plurality of electrodes (E), comprises, preferably consists of, a carbon-based ink and/or a conductive polymer-based ink, preferably the carbon-based ink comprising a conductive compound selected from the group consisting of graphene, graphite, nano-carbon-tubes and mixtures thereof, preferably the conductive polymer-based ink comprising a conductive compound selected from the group consisting of polyacetylene, polypyrrole, polyaniline and copolymers thereof, more preferably selected from the group consisting of poly(pyrrole)s (PPY), polyanilines (PANI), poly(thiophene)s (PT), poly(p-phenylene sulfide) (PPS), poly(p-phenylene) (PPP), Poly (acetylene) s (PAC), Poly (p-phenylene vinylene) (PPV), poly(3,4-ethylenedioxythiophene) (PEDOT), and mixtures thereof, most preferred conductive polymer-based ink comprising poly(3,4-ethylenedioxythiophene) polystyrene sulfonate (PEDOT:PSS). Advantageously, fast production is achieved whilst ensuring good compatibility with the end/final product unlike with the addition of foreign conductive elements like copper wires and the like, which are more prone to tearing the liquid impermeable substrate. Indeed, it is preferred in the disclosure herein to avoid metal containing inks (i.e. ink is free of metals) particularly due to the environmental impact to disposability.

The substrate (5) further comprises at least one insulating layer (80), as exemplified in Fig. 3, preferably being liquid impermeable, adhered thereto and sized to cover at least a portion of the conductive pattern (20) (typically said "at least portion" being proximal to a position where the data processing module (30) is connected to said substrate (5)). Said at least one insulating layer (80) is adapted to provide a seal and/or barrier to liquid and/or exudates from coming into contact with said portion of the conductive pattern (20), preferably wherein the conductors (C) and electrodes (E) remain exposed to liquids and/or exudates and are not covered by said at least one insulating layer (80). Advantageously, the at least one insulating layer (80) protects portions of the conductive pattern (20) to come into direct contact with exudates, limiting the risk of false positives, noise and even failure of detection. Preferably, the at least one insulating layer (80) is a nonconductive substrate such as a film typically comprising polyethylene, alternatively the at least one insulating layer (80) is in the form of a non-conductive adhesive (preferably a hotmelt adhesive) and/or non-conductive ink.

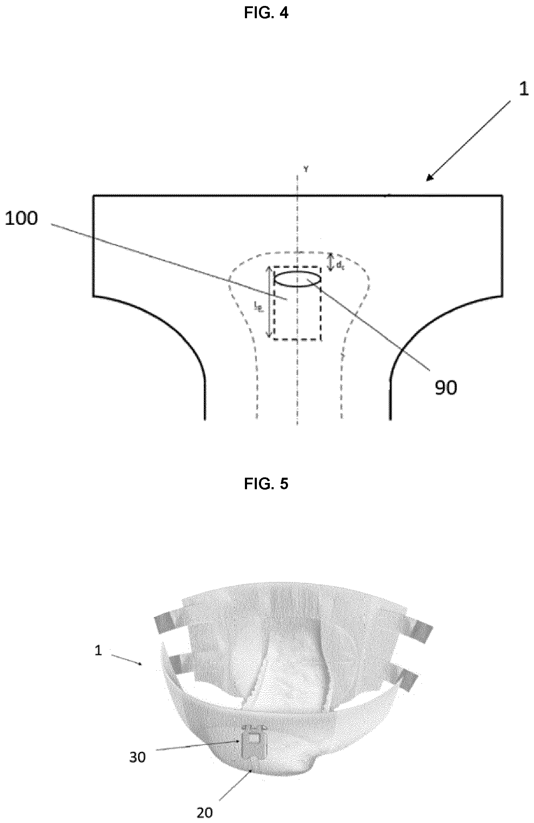

In a highly preferred embodiment, as depicted in Fig. 4, the substrate (5) comprises one or more slits (90) for insertion of the date processing module (30). In addition to this, a pocket (100) is formed between the first surface (21) and the at least one insulating layer (80) proximal to the first end (50), said pocket (100) being in fluid communication only with said slit(s) (90) and arranged to retain at least a portion of the data processing module (30) therein. This is beneficial, since an effective retaining pocket (100) is attained in a cheap and scalable manufacturing process allowing for manufacturing of such substrates (5) at high production speeds which still ensures a pocket (100) is formed to retain the module (30) in contact with the connection tracks (CT) whilst at the same time providing insulation against exudates as the substrate (5) becomes saturated.

Preferably, the pocket (100) is sized such that it matches the dimensions of a pocket insertion member of the data processing module (30), preferably a clip-on data processing module as exemplified in Figs. 5-7, which typically comprises terminals (T) such that said terminals (T) come into electrical communication with respective connection tracks (CT) by simply inserting the module (30) into the pocket (100) until a pocket dimension stops it from being inserted any further. Advantageously, this allows the caregiver to connect said module (30) without having to worry about particularly positioning the module (30) to ensure good electrical connection.

For insertion of the module (30), an opening cut (or slit) (90) can be created within the substrate (5). The opening cut (90) can be a simple straight line or it can be a shape that requires to remove the cut out trim during the manufacturing process. The opening cut (90) may be shaped in a way that it allows to identify easily the position for insertion of the module (30), which allows easy insertion and that can hold the module (30) securely in place after insertion.

In a preferred embodiment, the conductive pattern (20) extends symmetrically in a direction substantially parallel to the length L of the substrate (5) and in a direction substantially perpendicular thereto, defining a plurality of independent electrical circuits, and wherein combinations of said electrical circuits are in electrical communication by selectively addressing at least two of the conductors (C) with at least two corresponding terminals (T) of the data processing module (30). Preferably, the number of terminals (T) of the module (30) is at least the same as the number of connection tracks (CT).

Combinations of circuits defined by the conductive pattern (20) may be enabled by selectively addressing multiple combinations of at least pairs of connection tracks (CT) by multiple combinations of corresponding pairs of terminals (T). By selectively addressing multiple combinations of said plurality of connection tracks (CT) with corresponding terminals (T) of the data processing module (30), measurements of resistance, impedance and/or capacitance through the multiple electrical circuits defined by said conductive pattern (20) are also enabled.

Furthermore, portions of the conductive pattern (20) comprising a plurality of conductors (C) and a plurality of electrodes (E) may be designed in such a way that they are configured to undergo changes, preferably in its electrical properties, in presence of certain substances (such as stool and/or cellulose fibers with superabsorbent polymers saturated with urine, or the like). Thus, variations in the measured resistance, impedance and/or capacitance through selected electrical circuits defined by said conductors (C) and electrodes (C) allow to detect whether at least a portion of said circuit is in contact with wetness due to exposure to liquids and/or exudates. By selectively activating combinations of multiple circuits of the conductive pattern, it is possible to generate a mapping of the wetness along the absorbent article. Advantageously, accurate and automatic detection and monitoring of liquids and/or exudates in an efficient manner is enabled, ensuring scalable in in-line mass production at high speeds.

One may note that multiple combinations of available terminals (T) addressing corresponding connection tracks (CT) of the conductive pattern (20) can be chosen in order to detect specific and/or desired zones of the substrate (5) where wetness events are expected to occur.

According to an embodiment, the plurality of circuits defined by the conductive pattern (20) are circuits with a closed configuration and or alternatively with an open configuration. In addition to this or as an alternative, at least one of the plurality of circuits defined by the conductive pattern (20) is a circuit with an open configuration. Additionally or alternatively, the plurality of circuits defined by the conductive pattern have a configuration that enables space for applying glue lines when incorporated into an absorbent article (1). This is beneficial, since the electrical properties of different circuits are used to enhance sensitivity on detecting and monitoring wetness and they do not harm the circuits. Further advantageously, appropriate electrical connection is ensured and said glue lines do not harm the electrical properties of the circuits.

In a preferred embodiment, at least one of the plurality of circuits defined by the conductive pattern (20) is a circuit with a closed configuration, in order to ensure that a proper connectivity is achieved between the plurality of connection tracks (CT) of the conductive pattern (20) and corresponding terminals (T) of the clip-on module (30) when attached thereto.

In a preferred embodiment, the plurality of conductors (C) and the plurality of electrodes (E) are arranged to form at least one or more open electrical circuits, preferably between about 5 to about 50, even more preferably between about 10 to about 45, most preferably about 28. Advantageously, accurate and automatic detection and monitoring of liquids and/or exudates in an efficient manner is enabled, ensuring scalable in in-line mass production at high speeds.

In a preferred embodiment, the number of open electrical circuits may be higher in the front half (F) of the absorbent core (3) compared to the rear half (R) of the absorbent core (3). Advantageously, this allows for a higher sensitivity and higher accuracy in the front half of the absorbent core where the theoretical miction point is located.

In an embodiment, the conductive pattern (20) may have a shape selected from straight lines and/or curved lines. Advantageously, said arrangements may circumvent some defects on the printing process placing the conductive pattern onto the first surface of the substrate.

In an embodiment, the conductors (C) and electrodes (E) of the conductive pattern (20) may have a connected shape. The shapes may comprise, for example, solid or open rectangles and/or solid or open triangles and/or solid or open ellipses or a combination thereof. Preferably, conductors and electrodes may have a "T" shape.

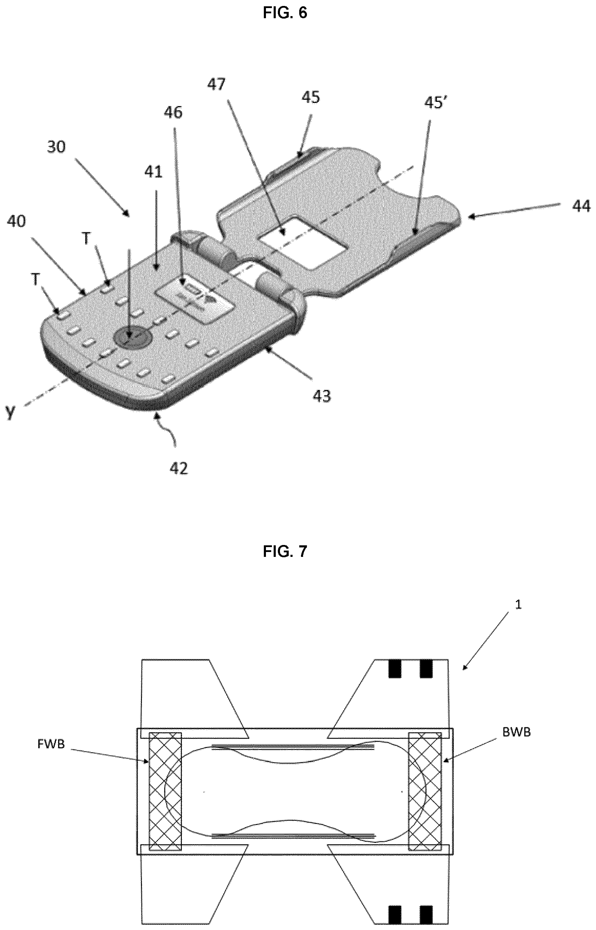

In an embodiment, the absorbent article (1) further comprises a front (FWB) and/or back waist barrier (BWB), as exemplified in Fig. 7, in which the front (FWB) and/or back waist barrier (BWB) optionally comprises at least a portion of said substrate (5) for monitoring and detecting the presence of wetness events therein and/or risk of exudate leakage therefrom. Advantageously, such presence of said substrate within or on the front and/or back waist barrier further aids in timely warning the caregiver to change the absorbent article of a patient before leakage occurs. The front and/or back waist barrier in itself already helps in preventing leakage however it is a direct indication that exudates have reached the longitudinal ends of the absorbent core.

### DATA PROCESSING MODULE

The data processing module (30) is configured to measure resistance, impedance and/or capacitance by multiple combinations of terminals (T) in order to generate said mapping of wetness. Additionally, the clip-on data processing module (30) can also be adapted to generate and provide a warning to the care giver when the measured electrical properties in at least one of the detection zones (Z) reaches a specific value, indicating that the absorbent article (1) must be replaced. Such a value can be preset and may be stored in a memory within the data processing module (30).

Data processing modules (30) for use herein are preferably of the re-fastenable and replaceable kind. This means that the modules (30) are adapted to be joined to an absorbent article (1) and subsequently decoupled therefrom once the absorbent article (1) is to be disposed. Thus, the modules (30) are adapted for multiple use and may be connected to a plurality of absorbent articles (1) in sequence (i.e. one after the other) and to the same or different patient/subject. The modules (30) may be cleaned and battery charged in between a plurality of absorbent article (1) changes. The net result is that an efficient, cost-effective and environment-compliant solution is provided.

In a preferred embodiment, the data processing module (30) is a clip-on data processing module which is in electrical communication with the conductive pattern (20) when connected at a position proximal to a first end (50) of the substrate (5) such to form at least one closed electrical circuit. This is beneficial, since the implementation of the inventive substrate into the final product is compatible with current production lines, which reduces operation costs compared to other solutions that require additional more complicated process steps and, furthermore, enables the use of the final product in a harmless manner for a wearer.

As exemplified in Fig. 5-6, the clip-on data processing module herein comprises a housing (40) which further comprises a front face (41), a back face (42), and side edges (43) connecting the front and back faces (41, 42) and disposed therebetween; and a clip element (44) rotatably connected to the housing (40), wherein the clip element (44) comprises one or more locking elements (45, 45') arranged to mechanically engage with at least a portion of the side edge (43) (preferably only a portion of said side edge typically said portion extending up to about half of the total depth of said side edge), when in a closed position, and wherein said clip element (44) can be rotated from an open position, where the locking elements (45, 45') do not mechanically engage with said side edge (43), to said closed position, and wherein said clip-on data processing module (30) is suitable for coupling to an absorbent article (1) and arranged such that when in said closed position at least one layer of said absorbent article (1) is interposed between said housing (40) and said clip element (44). Advantageously, this arrangement allows for a secure fit of the date processing module to the absorbent article in an intuitive and cost-effective way without risking to damage and/or tear the backsheet layer that is interposed between the clip element and the housing when the module is connected to the absorbent article, the interposed layer being beneficial to strongly adhere the clip-on data processing module to the absorbent article and ensure good electrical communication with the sensor tracks even when a subject moves around. It is undesirable that the locking elements of the clip element mechanically engage with a portion of the back face of the detection device as this could lead to higher chances of tearing.

The housing (40) comprises herein a battery, a processor, and a transmitter, and further comprises a plurality of conductive terminals (T) that are exposed on the front face (41) of said housing (40) and facing said clip element (44) when in the closed position, said conductive terminals (T) being arranged to come into contact with one or more conductive connection tracks (CT) positioned on the absorbent article (1), preferably a surface, typically a body facing surface, of the at least one layer of said absorbent article (1) interposed between the housing (40) and the clip element (44) when in the closed position, preferably said layer being a liquid impermeable backsheet of said absorbent article (1). Advantageously this allows for seamless and reliable connection with the conductive tracks of the absorbent article with limited risk of misalignment that would otherwise cause in exudate detection false measurement.

In an embodiment, the housing (40) further comprises a display (46) adapted to display information relating to a monitoring status, said display (46) being viewable from a garment facing side of the absorbent article (1) by a caregiver, said display (46) being positioned on the front face (41) of the housing (40). Advantageously by placing all electronics (including the screen on the housing, it allows for easy removal of the clip element for cleaning and/or replacement in case of damage without having to replace the electronic components hence resulting in a more cost-effective device in the longterm.

Preferably, the clip element (44) comprises a cut-out or window (47) sized such that the display (46) may protrude or be seen therethrough when in the closed position, preferably such that when in the closed position the display (46) can be viewed by a caregiver. This arrangement advantageously allows the display to be contained in the housing and the clip element to securely fasten thereto whilst not obstructing the visibility of the screen of the display.

The absorbent article comprises a pocket (100) sized to accommodate the housing (40) of the clip-on data processing module (30) therein and to expose at least a portion thereof. In an embodiment said portion corresponds substantially to the size of the display (46). Advantageously, the pocket is sized precisely to accommodate the detection devices herein so as to make it as intuitive and simple for the caregivers to assemble and manipulate.

### METHOD FOR WETNESS MONITORING

The present invention provides for a method for monitoring wetness events in an absorbent article (1). The absorbent article (1) comprises a liquid permeable topsheet (2), a substantially liquid impermeable backsheet (4), an absorbent core (3) positioned between said topsheet (2) and backsheet (4) and a substrate (5) adapted for incorporation into the absorbent article (1). The substrate (5) comprises a plurality of conductors (C) and a plurality of electrodes (E) arranged to undergo a change in electrical properties upon one or more wetness events. Upon such a wetness event, the method comprises the step of determining the number of conductors triggered by said wetness event to cumulatively determine the number of activated electrodes (N). This cumulative number of activated electrodes (N) is compared to a threshold value (Nt) wherein an alarm is triggered when N is equal to or greater than (Nt). Advantageously, this method for monitoring wetness provides for an accurate and effective warning system allowing patients to get changed at the right time.

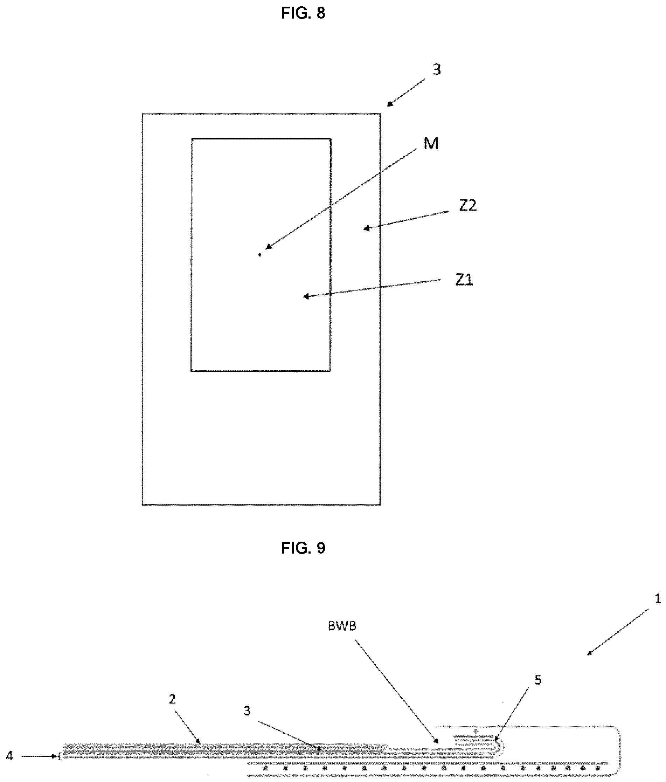

In a preferred embodiment, as exemplified in Fig. 8, the absorbent core (3) of the absorbent article (1) is divided in at least a first eccentric detection zone (Z1) and at least a second eccentric detection zone (Z2) wherein the second eccentric detection zone (Z2) is located closer to the periphery of the absorbent core (3) than the at least first eccentric detection zone (Z1). Preferably, the first eccentric detection zone (Z1) is centered around a theoretical miction point (M) and each of the at least first and second eccentric detection zones (Z1, Z2) comprise a portion of said plurality of conductors (C) and a portion of said plurality of electrodes (E). Upon a wetness event, the method comprises the step of determining the number of conductors (C) triggered by said wetness event in the at least first eccentric detection zone (Z1) to cumulatively determine the number of activated electrodes (N1) in said at least first eccentric detection zone (Z1) and determining the number of conductors (C) in the at least second eccentric detection zone (Z2) to cumulatively determine the number of activated electrodes (N2) in said at least second eccentric detection zone (Z2). Subsequently, the cumulative number of activated electrodes (N1) in said at least first eccentric detection zone (Z1) is compared to a first threshold value (Nt,1) and the cumulative number of activated electrodes (N2) in said at least second eccentric detection zone (Z2) is compared to a second threshold value (Nt,2) wherein an alarm is triggered when N2 is equal to or greater than (Nt,2). In an alternative embodiment, the at least first and second detection zones (Z1, Z2) are concentric about the centre point (CP) of the absorbent core (3), the centre point (CP) located at the centre in width direction (x) and longitudinal direction (y) of the absorbent core (3). Advantageously, this method for monitoring wetness provides for a more accurate and effective warning system allowing patients to get changed at the right time but not too soon. In this way, absorbent articles are used effectively without negatively impacting the patients but positively impacting the number of absorbent articles used.

In a further embodiment, an alarm is triggered when the number of activated electrodes (N2) in said at least second eccentric or concentric detection zone (Z2) is smaller than said second threshold value (Nt,2) and the number of activated electrodes (N1) in said at least first eccentric or concentric detection zone (Z1) is equal to or greater than (Nt,1). Advantageously, this provides for a timely alarm when wetness is detected in a peripheral zone of the absorbent core. This method allows for a timely change of patients before leakage occurs. Furthermore, it prevents false alarms in cases where a false detection occurs in one of the peripheral zones of the absorbent core.

In an embodiment, the at least first and second eccentric or concentric detection zones (Z1, Z2) have a substantially ellipsoidal or rectangular shape, as exemplified in Fig.8. Preferably, the shape of the eccentric or concentric detection zones follow the shape of the outline of the absorbent core (3). Advantageously, the eccentric detection zones can be tailored to the shape of the absorbent core and/or absorbent article which provides for a more effective wetness monitoring method.

In a preferred embodiment, the at least first and second threshold values (Nt,1; Nt,2) appointed to the at least first and second eccentric or concentric detection zone (Z1, Z2) are based on the probability of leakage. It is known that certain zones provide for a higher probability of leakage than other zones. For instance, detection of wetness in a peripheral zone would result in a higher probability of leakage than a zone in the central area of the absorbent core. Inherent with the use of such absorbent articles, eventually one or more wetness and/or exudate events will occur and thus need for change of the absorbent article arises. Particularly in home care and/or institutions, handling elderly with incontinence problems, knowing when to change the diaper/pant of a patient is of the utmost importance. Indeed, changing the diapers too early results in unnecessary cost and waste and changing too late results in further cleaning costs and time, and uncomfort or discomfort to the patient. This problem is exacerbated by motion, indeed taking into account that a subject wearing the article may move into different positions the saturation thereof and/or risk of leakage may vary considerably based on the position and time the subject remains in that position during and/or after one or more wetness/exudate events.

In a preferred embodiment, an alarm is triggered when the cumulative number of activated electrodes (N2) in said second eccentric or concentric detection zone (Z2) is smaller than the second threshold value (Nt,2) and the cumulative number of activated electrodes (N1) in said first eccentric or concentric detection zone (Z1) is equal to or higher than the first threshold value (Nt,1). Advantageously, false positive triggers can be eliminated and reduced. For example, an activated electrode in the second eccentric detection zone not related to a wetness event does not lead to an alarm if there is not a certain amount of wetness present in the first eccentric detection zone.

In an embodiment, the threshold value(s) (Nt; Nt,1; Nt,2) are settable with an API. Advantageously, such an API simplifies programming by abstracting the underlying implementation and only exposing objects or actions the developer needs.

In an embodiment, as exemplified in Fig. 6, the alarm is provided by an output element or display element (46) within the housing (40) of a data processing module (30) or attached to the data processing module (30) or provided in a separate unit. In a further embodiment, the alarm includes a visual output and/or audible output and/or a tactile output provided by an output element or display element (46). Advantageously, such alarms at these locations provide for clear indications to caregivers on the status of the wetness monitoring system for each patient.

In an embodiment, as exemplified in Fig. 9, the absorbent article (1) further comprises a front (FWB) and/or back waist barrier (BWB) in which the front and/or back waist barrier (BWB) comprises at least a portion of said substrate (5) for monitoring and detecting the presence of wetness events therein and/or risk of exudate leakage therefrom. Said portion comprising a plurality of conductors (C) and a plurality of electrodes (E) and wherein said portion of said substrate (5) comprises at least a portion of a third detection zone (Z3). Preferably, said third detection zone is entirely located within or on said portion of said substrate in the front (FWB) and/or back waist barrier (BWB). Advantageously, such presence of said substrate within or on the front and/or back waist barrier further aids in timely warning the caregiver to change the absorbent article of a patient before leakage occurs. The front and/or back waist barrier in itself already helps in preventing leakage however it is a direct indication that exudates have reached the longitudinal ends of the absorbent core.

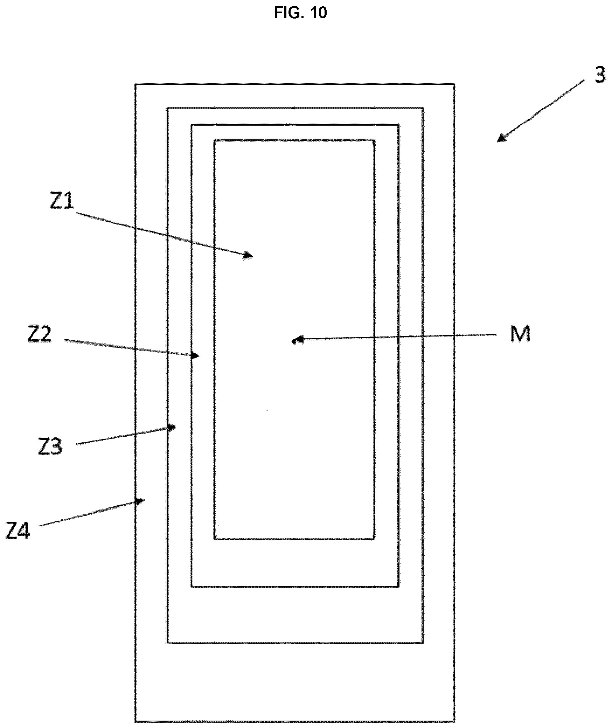

In an embodiment, as exemplified in Fig. 10, the absorbent core (3) is divided into at least 4 eccentric detection zones (Z1, Z2, Z3, Z4) wherein the fourth eccentric detection zone is located closest to the periphery of the absorbent core (3) and the first eccentric detection zone located farthest away from the periphery of the absorbent core (3). Preferably, the first eccentric detection zone (Z1) is centered around a theoretical miction point (M). Each of the zones comprises a portion of said plurality of conductors (C) and a portion of said plurality of electrodes (E). Upon a wetness event, the method comprises the step of determining the number of conductors (C) triggered by said wetness event in the at least first eccentric detection zone (Z1) to cumulatively determine the number of activated electrodes (N1) in said at least first eccentric detection zone (Z1), in the at least second eccentric detection zone (Z2) to cumulatively determine the number of activated electrodes (N2) in said at least second eccentric detection zone (Z2), in the at least third eccentric detection zone (Z3) to cumulatively determine the number of activated electrodes (N3) in said at least third eccentric detection zone (Z3) and in the at least fourth eccentric detection zone (Z4) to cumulatively determine the number of activated electrodes (N4) in said at least fourth eccentric detection zone (Z4). Subsequently, the cumulative number of activated electrodes (N1) in said at least first eccentric detection zone (Z1) is compared to a first threshold value (Nt,1), the cumulative number of activated electrodes (N2) in said at least second eccentric detection zone (Z2) is compared to a second threshold value (Nt,2), the cumulative number of activated electrodes (N3) in said at least third eccentric detection zone (Z3) is compared to a third threshold value (Nt,3), the cumulative number of activated electrodes (N4) in said at least fourth eccentric detection zone (Z4) is compared to a fourth threshold value (Nt,4). An alarm is triggered being at least a visual color signal according to 4 different levels representing a traffic light system. This traffic light system provides for the product status in relation to product saturation and leakage probability. An example of such an alarm notification system is provided below:
Example of traffic light system:
- Blue: No wetness detected = no probability of leakage → no action of the caregiver required
- Green: No probability of leakage unless the product is incorrectly applied → no immediate action required
- **Orange**: Still a normal risk of leakage but more than blue and green → no immediate action required
- **Red:** High probability of leakage → it is recommended to change the product as soon as possible

Example of triggers according to the ,above mentioned, exemplary traffic light system:
A red color signal is provided in the following occasions:
- $N 4 > = Nt , 4$
- $Nt , 4 > N 4 > = 1 and N 3 > 0$

An orange color signal is provided in the following occasions:
- $N 4 = 0 and N 3 > = Nt , 3$
- $N 4 = 0 and Nt , 3 > N 3 > = 1 and N 2 > 0$

A green color signal is provided in the following occasions:
- $N 4 = 0 and N 3 = 0 and N 2 > = Nt , 2$
- $N 4 = 0 and N 3 = 0 and Nt , 2 > N 2 > = 1 and N 1 > 0$

A blue color signal is provided in the following occasions:
- $N 4 = 0 and N 3 = 0 and N 2 = 0$

Advantageously, this method for monitoring wetness provides for a more accurate and effective warning system allowing patients to get changed at the right time but not too soon. In this way, absorbent articles are used effectively without negatively impacting the patients but positively impacting the number of absorbent articles used.

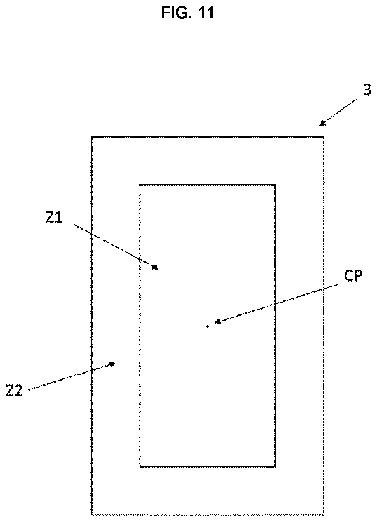

In an alternative embodiment, as exemplified in Fig.11, the at least first and second detection zones are concentric about the centre point (CP) of the absorbent core, the centre point located at the centre in width direction and longitudinal direction of the absorbent core. Advantageously, this provides for a flexible product in terms of wearer profile, being suitable for multiple wearer types.

### THE PROCESS

The disclosure herein further contemplates a method for making an absorbent article (1) comprising the steps of: providing a liquid impermeable backsheet (4) and applying a conductive pattern (20) as described herein to a first surface (21) thereof; providing and adhering at least one insulating layer (80) to said backsheet (4), said insulating layer (80) being sized and positioned to cover at least a portion of the conductors (C) of the conductive pattern (20), to provide a laminated substrate; providing an absorbent core (3) comprising absorbent material; providing a liquid permeable topsheet (2); and sandwiching the absorbent core (3) between said laminated substrate and said topsheet (2).

The method further comprises the step of providing a slit (90), additionally or alternatively a pocket (100), on the backsheet (4) enabling an electrically conductive portion of a clip-on data processing module (30) to selectively come in electrical communication with the connection tracks (CT) of the conductive pattern (20).

Preferably, the conductive pattern (20) is in the form of an electrically conductive ink and the application step comprises the printing thereof onto the backsheet (4). Moreover, the process generally comprises the step of detecting registered marks (70) to trigger accurate cutting of the substrate (5) (or the assembled/laminated continuous absorbent article 100) into a plurality discrete absorbent articles (1).

In a preferred embodiment the at least one insulating layer (80) is formed to have a width w, taken along an axis perpendicular to the longitudinal axis (y-y), being less than a width W of the backsheet (4) or substrate (5). Preferably, the width w is less than 0.5W, more preferably from 0.05W to 0.35W, even more preferably from 0.08W to 0.30W, most preferably from 0.1W to 0.2W.

In an embodiment, the layers referred to herein above (i.e. at least the backsheet (4) and the at least one insulating layer (80)) are in the form of continuous webs and/or films that are first printed with conductive ink in the conductive pattern (20) described above prior to being laminated together and subsequently cut into individual absorbent articles (1).

In an embodiment, the at least one insulating layer (80) is adhered to the backsheet (4) by an adhesive and/or mechanical bonding, wherein the mechanical bonding is preferably selected from ultrasonic bonding, thermal bonding, and combinations thereof. In a preferred embodiment, the adhesive and/or mechanical bonding is applied across a length and width of the at least one insulating layer (80) in an effective amount such that bonding is achieved with the backsheet (4) and a liquid impermeable seal is formed providing a barrier to exudates expelled by a subject (when wearing the absorbent article (1)) from coming into direct contact with at least a portion of the conductive pattern (20), preferably wherein at a location proximal to the first end (50) of the backsheet (4), where the clip-on module (30) is to be connected the adhesive and is present only outboard of the connection tracks (CT) and not therebetween such to form a pocket (100) for receiving an electrically conducting portion of said module (30).

In a preferred embodiment, monitoring and detecting the presence of wetness at different zones of the core of the absorbent article (1) is enabled by measuring resistance, impedance and/or capacitance of multiple circuit configurations defined by the conductive pattern (20) and selectively addressed by corresponding terminals (T) of the clip-on module (30).

## Claims

1. Method for monitoring wetness events in an absorbent article (1) comprising the steps of:
a. Providing an absorbent article (1) comprising a liquid permeable topsheet (2), a substantially liquid impermeable backsheet (4), an absorbent core (3) positioned between said topsheet (2) and backsheet (4) and a substrate (5) adapted for incorporation into the absorbent article (1) comprising a plurality of conductors (C) and a plurality of electrodes (E) arranged to undergo a change in electrical properties upon one or more wetness events;
b. Wherein the method, upon a wetness event, comprises the step of determining the number of conductors triggered by said wetness event to cumulatively determine the number of activated electrodes (N);
c. Comparing said cumulative number of activated electrodes (N) to a threshold value (Nt);
d. Triggering an alarm when N is equal to or greater than (Nt).

2. Method for monitoring wetness events in an absorbent article (1), according to claim 1, wherein the absorbent core (3) is divided in at least a first eccentric detection zone (Z1) and at least a second eccentric detection zone (Z2), the second eccentric detection zone (Z2) located closer to the periphery of the absorbent core (3) than the at least first eccentric detection zone (Z1), preferably centered around a theoretical miction point (M) and wherein each of said eccentric detection zones comprises a portion of said plurality of conductors (C) and a portion of said plurality of electrodes (E);
b. wherein the method, upon a wetness event, comprises the step of determining the number of conductors triggered by said wetness event in an at least first eccentric detection zone (Z1) to cumulatively determine the number of activated electrodes (N1) in said at least first eccentric detection zone (Z1); and determining the number of conductors triggered by said wetness event in an at least second eccentric detection zone (Z2) to cumulatively determine the number of activated electrodes (N2) in said at least second eccentric detection zone (Z2);
c. Comparing said cumulative number of activated electrodes (N1) in said at least first eccentric detection zone (Z1) to a first threshold value (Nt,1) and said cumulative number of activated electrodes (N2) in said at least second eccentric detection zone (Z2) to a second threshold value (Nt, 2);
d. Triggering an alarm when N2 is equal to or greater than (Nt,2).

3. Method for monitoring wetness events in an absorbent article (1), according to any of the preceding claims, wherein said substantially liquid impermeable backsheet (4) comprises said substrate (5).

4. Method for monitoring wetness events in an absorbent article (1) according to any of claims 2 to 3, wherein a threshold value (Nt,1; Nt,2) is appointed to each eccentric detection zone (Z1, Z2) based on probability of leakage.

5. Method for monitoring wetness events in an absorbent article (1) according to any of claims 2 to 4, wherein an alarm is triggered when N2 is smaller than (Nt,2) and N1 is equal to or greater than (Nt, 1).

6. Method for monitoring wetness events in an absorbent article (1) according to any of claims 2 to 5, wherein the at least 2 eccentric detection zones (Z1, Z2) have a substantially ellipsoidal and/or rectangular shape.

7. Method for monitoring wetness events in an absorbent article (1) according to any of the preceding claims, wherein at least a portion of said plurality of conductors (C) is connected to a data processing module (6), preferably a clip-on data processing module.

8. Method for monitoring wetness events in an absorbent article (1) according to any of the preceding claims, wherein said plurality of conductors (C) and said plurality of electrodes (E) are arranged in one or more open electrical circuits (EC), preferably between about 5 to about 50, even more preferably between about 10 to about 45, most preferably about 28.

9. Method for monitoring wetness events in an absorbent article (1) according to any of the preceding claims, wherein the threshold value(s) (Nt; Nt,1; Nt,2) is settable with an API (7).

10. Method for monitoring wetness events in an absorbent article (1) according to any of the preceding claims, wherein the number of open electrical circuits (EC) is higher in the front half (F) of the absorbent article (1) compared to the rear half (R) of the absorbent article (1).

11. Method for monitoring wetness events in an absorbent article (1) according to any of the preceding claims, wherein the plurality of conductors (C) and the plurality of electrodes (E) are printed with a conductive ink, preferably wherein the conductive ink is a carbon-based ink and/or a conductive polymer-based ink.

12. Method for monitoring wetness events in an absorbent article (1) according to any of the preceding claims, wherein the alarm is provided by an output element or display element (8) within the housing (9) of a detection device (10) or attached to the detection device (10) or provided in a separate unit (11).

13. Method for monitoring wetness events in an absorbent article (1) according to claim 12, wherein the alarm includes a visual output and/or an audible output and/or a tactile output provided by the output element or display element (8).

14. Method for monitoring wetness events in an absorbent article (1) according to any of the preceding claims, wherein the absorbent article (1) further comprises a front (FWB) and/or back waist barrier (BWB) in which the front (FWB) and/or back waist barrier (BWB) optionally comprises at least a portion of said substrate (5).

15. Method for monitoring wetness events in an absorbent article () according to claim 14, wherein said portion of said substrate (5) comprises a plurality of conductors and a plurality of electrodes and wherein said portion of said substrate (5) comprises a portion of an at least third detection zone (Z3);
b. wherein the method further, upon a wetness event, comprises the step of determining the number of conductors triggered by said wetness event in an at least third detection zone (Z3) to cumulatively determine the number of activated electrodes (N3) in said at least third detection zone (Z3);
c. Comparing said cumulative number of activated electrodes (N3) in said at least third eccentric detection zone (Z3) to a third threshold value (Nt,3);
d. Triggering an alarm when N3 is equal to or greater than (Nt,3).

## Patentansprüche

1. Verfahren zur Überwachung von Nässeereignissen in einem absorbierenden Artikel (1), umfassend die Schritte:
a. Bereitstellen eines absorbierenden Artikels (1), umfassend eine flüssigkeitsdurchlässige Oberschicht (2), eine im Wesentlichen flüssigkeitsundurchlässige Unterschicht (4), einen zwischen der besagten Oberschicht (2) und der Unterschicht (4) positionierten absorbierenden Kern (3) und ein Substrat (5), das zur Einarbeitung in den absorbierenden Artikel (1) angepasst ist und eine Vielzahl von Leitern (C) und eine Vielzahl von Elektroden (E) umfasst, die so angeordnet sind, dass sie bei einem oder mehreren Nässeereignissen eine Änderung der elektrischen Eigenschaften erfahren;
b. Wobei das Verfahren bei einem Nässeereignis den Schritt des Bestimmens der Anzahl der durch das besagte Nässeereignis ausgelösten Leiter umfasst, um die Anzahl der aktivierten Elektroden (N) kumulativ zu bestimmen;
c. Vergleichen der besagten kumulativen Anzahl aktivierter Elektroden (N) mit einem Schwellenwert (Nt);
d. Auslösen eines Alarms, wenn N gleich oder größer als (Nt) ist.

2. Verfahren zur Überwachung von Nässeereignissen in einem absorbierenden Artikel (1) nach Anspruch 1, wobei der absorbierende Kern (3) in mindestens eine erste exzentrische Detektionszone (Z1) und mindestens eine zweite exzentrische Detektionszone (Z2) unterteilt ist, wobei die zweite exzentrische Detektionszone (Z2) näher an der Peripherie des absorbierenden Kerns (3) liegt als die mindestens erste exzentrische Detektionszone (Z1), vorzugsweise um einen theoretischen Miktionspunkt (M) zentriert ist, und wobei jede der besagten exzentrischen Detektionszonen einen Teil der besagten Vielzahl von Leitern (C) und einen Teil der besagten Vielzahl von Elektroden (E) umfasst;
b. wobei das Verfahren bei einem Nässeereignis den Schritt des Bestimmens der Anzahl der durch das besagte Nässeereignis ausgelösten Leiter in mindestens einer ersten exzentrischen Detektionszone (Z1) umfasst, um die Anzahl der aktivierten Elektroden (N1) in der besagten mindestens ersten exzentrischen Detektionszone (Z1) kumulativ zu bestimmen; und des Bestimmens der Anzahl der durch das besagte Nässeereignis ausgelösten Leiter in mindestens einer zweiten exzentrischen Detektionszone (Z2), um die Anzahl der aktivierten Elektroden (N2) in der besagten mindestens zweiten exzentrischen Detektionszone (Z2) kumulativ zu bestimmen;
c. Vergleichen der besagten kumulativen Anzahl aktivierter Elektroden (N1) in der besagten mindestens ersten exzentrischen Detektionszone (Z1) mit einem ersten Schwellenwert (Nt,1) und der besagten kumulativen Anzahl aktivierter Elektroden (N2) in der besagten mindestens zweiten exzentrischen Detektionszone (Z2) mit einem zweiten Schwellenwert (Nt,2);
d. Auslösen eines Alarms, wenn N2 gleich oder größer als (Nt,2) ist.

3. Verfahren zur Überwachung von Nässeereignissen in einem absorbierenden Artikel (1) nach einem der vorhergehenden Ansprüche, wobei die besagte im Wesentlichen flüssigkeitsundurchlässige Unterschicht (4) das besagte Substrat (5) umfasst.

4. Verfahren zur Überwachung von Nässeereignissen in einem absorbierenden Artikel (1) nach einem der Ansprüche 2 bis 3, wobei jeder exzentrischen Detektionszone (Z1, Z2) ein Schwellenwert (Nt,1 ; Nt,2) basierend auf der Auslaufwahrscheinlichkeit zugewiesen wird.

5. Verfahren zur Überwachung von Nässeereignissen in einem absorbierenden Artikel (1) nach einem der Ansprüche 2 bis 4, wobei ein Alarm ausgelöst wird, wenn N2 kleiner als (Nt,2) ist und N1 gleich oder größer als (Nt,1) ist.

6. Verfahren zur Überwachung von Nässeereignissen in einem absorbierenden Artikel (1) nach einem der Ansprüche 2 bis 5, wobei die mindestens 2 exzentrischen Detektionszonen (Z1, Z2) eine im Wesentlichen ellipsoide und/oder rechteckige Form haben.

7. Verfahren zur Überwachung von Nässeereignissen in einem absorbierenden Artikel (1) nach einem der vorhergehenden Ansprüche, wobei mindestens ein Teil der besagten Vielzahl von Leitern (C) mit einem Datenverarbeitungsmodul (6), vorzugsweise einem aufsteckbaren Datenverarbeitungsmodul, verbunden ist.

8. Verfahren zur Überwachung von Nässeereignissen in einem absorbierenden Artikel (1) nach einem der vorhergehenden Ansprüche, wobei die besagte Vielzahl von Leitern (C) und die besagte Vielzahl von Elektroden (E) in einem oder mehreren offenen Stromkreisen (EC) angeordnet sind, vorzugsweise zwischen etwa 5 bis etwa 50, noch bevorzugter zwischen etwa 10 bis etwa 45, am meisten bevorzugt etwa 28.

9. Verfahren zur Überwachung von Nässeereignissen in einem absorbierenden Artikel (1) nach einem der vorhergehenden Ansprüche, wobei der/die Schwellenwert(e) (Nt; Nt,1; Nt,2) mit einer API (7) einstellbar ist.

10. Verfahren zur Überwachung von Nässeereignissen in einem absorbierenden Artikel (1) nach einem der vorhergehenden Ansprüche, wobei die Anzahl der offenen Stromkreise (EC) in der vorderen Hälfte (F) des absorbierenden Artikels (1) im Vergleich zur hinteren Hälfte (R) des absorbierenden Artikels (1) höher ist.

11. Verfahren zur Überwachung von Nässeereignissen in einem absorbierenden Artikel (1) nach einem der vorhergehenden Ansprüche, wobei die Vielzahl der Leiter (C) und die Vielzahl der Elektroden (E) mit einer leitfähigen Tinte bedruckt sind, wobei die leitfähige Tinte vorzugsweise eine Tinte auf Kohlenstoffbasis und/oder eine Tinte auf Basis eines leitfähigen Polymers ist.

12. Verfahren zur Überwachung von Nässeereignissen in einem absorbierenden Artikel (1) nach einem der vorhergehenden Ansprüche, wobei der Alarm durch ein Ausgabeelement oder Anzeigeelement (8) innerhalb des Gehäuses (9) einer Detektionsvorrichtung (10) oder an der Detektionsvorrichtung (10) angebracht oder in einer separaten Einheit (11) bereitgestellt wird.

13. Verfahren zur Überwachung von Nässeereignissen in einem absorbierenden Artikel (1) nach Anspruch 12, wobei der Alarm eine visuelle Ausgabe und/oder eine hörbare Ausgabe und/oder eine taktile Ausgabe umfasst, die durch das Ausgabeelement oder Anzeigeelement (8) bereitgestellt wird.

14. Verfahren zur Überwachung von Nässeereignissen in einem absorbierenden Artikel (1) nach einem der vorhergehenden Ansprüche, wobei der absorbierende Artikel (1) ferner eine vordere (FWB) und/oder hintere Taillenbarriere (BWB) umfasst, wobei die vordere (FWB) und/oder hintere Taillenbarriere (BWB) optional mindestens einen Teil des besagten Substrats (5) umfasst.

15. Verfahren zur Überwachung von Nässeereignissen in einem absorbierenden Artikel () nach Anspruch 14, wobei der besagte Teil des besagten Substrats (5) eine Vielzahl von Leitern und eine Vielzahl von Elektroden umfasst und wobei der besagte Teil des besagten Substrats (5) einen Teil von mindestens einer dritten Detektionszone (Z3) umfasst;
b. wobei das Verfahren ferner bei einem Nässeereignis den Schritt des Bestimmens der Anzahl der durch das besagte Nässeereignis ausgelösten Leiter in mindestens einer dritten Detektionszone (Z3) umfasst, um die Anzahl der aktivierten Elektroden (N3) in der besagten mindestens dritten Detektionszone (Z3) kumulativ zu bestimmen;
c. Vergleichen der besagten kumulativen Anzahl aktivierter Elektroden (N3) in der besagten mindestens dritten exzentrischen Detektionszone (Z3) mit einem dritten Schwellenwert (Nt,3);
d. Auslösen eines Alarms, wenn N3 gleich oder größer als (Nt,3) ist.

## Revendications

1. Procédé de surveillance d'événements d'humidité dans un article absorbant (1) comprenant les étapes consistant à :
a. Fournir un article absorbant (1) comprenant une feuille de dessus perméable aux liquides (2), une feuille de fond sensiblement imperméable aux liquides (4), une âme absorbante (3) positionnée entre ladite feuille de dessus (2) et ladite feuille de fond (4) et un substrat (5) adapté pour être incorporé dans l'article absorbant (1) comprenant une pluralité de conducteurs (C) et une pluralité d'électrodes (E) agencées pour subir un changement de propriétés électriques lors d'un ou plusieurs événements d'humidité ;
b. Dans lequel le procédé, lors d'un événement d'humidité, comprend l'étape consistant à déterminer le nombre de conducteurs déclenchés par ledit événement d'humidité pour déterminer cumulativement le nombre d'électrodes activées (N) ;
c. Comparer ledit nombre cumulé d'électrodes activées (N) à une valeur de seuil (Nt) ;
d. Déclencher une alarme lorsque N est égal ou supérieur à (Nt).

2. Procédé de surveillance d'événements d'humidité dans un article absorbant (1), selon la revendication 1, dans lequel l'âme absorbante (3) est divisée en au moins une première zone de détection excentrique (Z1) et au moins une deuxième zone de détection excentrique (Z2), la deuxième zone de détection excentrique (Z2) étant située plus près de la périphérie de l'âme absorbante (3) que l'au moins une première zone de détection excentrique (Z1), de préférence centrée autour d'un point de miction théorique (M) et dans lequel chacune desdites zones de détection excentriques comprend une partie de ladite pluralité de conducteurs (C) et une partie de ladite pluralité d'électrodes (E) ;
b. dans lequel le procédé, lors d'un événement d'humidité, comprend l'étape de détermination du nombre de conducteurs déclenchés par ledit événement d'humidité dans au moins une première zone de détection excentrique (Z1) pour déterminer cumulativement le nombre d'électrodes activées (N1) dans ladite au moins une première zone de détection excentrique (Z1) ; et la détermination du nombre de conducteurs déclenchés par ledit événement d'humidité dans au moins une deuxième zone de détection excentrique (Z2) pour déterminer cumulativement le nombre d'électrodes activées (N2) dans ladite au moins une deuxième zone de détection excentrique (Z2) ;
c. Comparer ledit nombre cumulé d'électrodes activées (N1) dans ladite au moins une première zone de détection excentrique (Z1) à une première valeur de seuil (Nt,1) et ledit nombre cumulé d'électrodes activées (N2) dans ladite au moins une deuxième zone de détection excentrique (Z2) à une deuxième valeur de seuil (Nt, 2) ;
d. Déclencher une alarme lorsque N2 est égal ou supérieur à (Nt,2).

3. Procédé de surveillance d'événements d'humidité dans un article absorbant (1), selon l'une quelconque des revendications précédentes, dans lequel ladite feuille de fond sensiblement imperméable aux liquides (4) comprend ledit substrat (5).

4. Procédé de surveillance d'événements d'humidité dans un article absorbant (1) selon l'une quelconque des revendications 2 à 3, dans lequel une valeur de seuil (Nt,1 ; Nt,2) est attribuée à chaque zone de détection excentrique (Z1, Z2) sur la base de la probabilité de fuite.

5. Procédé de surveillance d'événements d'humidité dans un article absorbant (1) selon l'une quelconque des revendications 2 à 4, dans lequel une alarme est déclenchée lorsque N2 est inférieur à (Nt,2) et N1 est égal ou supérieur à (Nt,1).

6. Procédé de surveillance d'événements d'humidité dans un article absorbant (1) selon l'une quelconque des revendications 2 à 5, dans lequel les au moins 2 zones de détection excentriques (Z1, Z2) ont une forme sensiblement ellipsoïdale et/ou rectangulaire.

7. Procédé de surveillance d'événements d'humidité dans un article absorbant (1) selon l'une quelconque des revendications précédentes, dans lequel au moins une partie de ladite pluralité de conducteurs (C) est connectée à un module de traitement de données (6), de préférence un module de traitement de données à clipser.

8. Procédé de surveillance d'événements d'humidité dans un article absorbant (1) selon l'une quelconque des revendications précédentes, dans lequel ladite pluralité de conducteurs (C) et ladite pluralité d'électrodes (E) sont agencées en un ou plusieurs circuits électriques ouverts (EC), de préférence entre environ 5 et environ 50, encore plus préférablement entre environ 10 et environ 45, le plus préférablement environ 28.

9. Procédé de surveillance d'événements d'humidité dans un article absorbant (1) selon l'une quelconque des revendications précédentes, dans lequel la ou les valeurs de seuil (Nt ; Nt,1 ; Nt,2) sont réglables avec une API (7).

10. Procédé de surveillance d'événements d'humidité dans un article absorbant (1) selon l'une quelconque des revendications précédentes, dans lequel le nombre de circuits électriques ouverts (EC) est plus élevé dans la moitié avant (F) de l'article absorbant (1) que dans la moitié arrière (R) de l'article absorbant (1).

11. Procédé de surveillance d'événements d'humidité dans un article absorbant (1) selon l'une quelconque des revendications précédentes, dans lequel la pluralité de conducteurs (C) et la pluralité d'électrodes (E) sont imprimées avec une encre conductrice, de préférence dans lequel l'encre conductrice est une encre à base de carbone et/ou une encre à base de polymère conducteur.

12. Procédé de surveillance d'événements d'humidité dans un article absorbant (1) selon l'une quelconque des revendications précédentes, dans lequel l'alarme est fournie par un élément de sortie ou un élément d'affichage (8) à l'intérieur du boîtier (9) d'un dispositif de détection (10) ou fixée au dispositif de détection (10) ou fournie dans une unité séparée (11).

13. Procédé de surveillance d'événements d'humidité dans un article absorbant (1) selon la revendication 12, dans lequel l'alarme comprend une sortie visuelle et/ou une sortie audible et/ou une sortie tactile fournie par l'élément de sortie ou l'élément d'affichage (8).

14. Procédé de surveillance d'événements d'humidité dans un article absorbant (1) selon l'une quelconque des revendications précédentes, dans lequel l'article absorbant (1) comprend en outre une barrière de taille avant (FWB) et/ou une barrière de taille arrière (BWB) dans lesquelles la barrière de taille avant (FWB) et/ou la barrière de taille arrière (BWB) comprennent facultativement au moins une partie dudit substrat (5).

15. Procédé de surveillance d'événements d'humidité dans un article absorbant () selon la revendication 14, dans lequel ladite partie dudit substrat (5) comprend une pluralité de conducteurs et une pluralité d'électrodes et dans lequel ladite partie dudit substrat (5) comprend une partie d'au moins une troisième zone de détection (Z3) ;
b. dans lequel le procédé comprend en outre, lors d'un événement d'humidité, l'étape consistant à déterminer le nombre de conducteurs déclenchés par ledit événement d'humidité dans au moins une troisième zone de détection (Z3) pour déterminer cumulativement le nombre d'électrodes activées (N3) dans ladite au moins une troisième zone de détection (Z3) ;
c. Comparer ledit nombre cumulé d'électrodes activées (N3) dans ladite au moins une troisième zone de détection excentrique (Z3) à une troisième valeur de seuil (Nt,3) ;
d. Déclencher une alarme lorsque N3 est égal ou supérieur à (Nt,3).
